# EUROPEAN PATENT APPLICATION

(11) **EP 2 458 043 A1**
(43) Date of publication of application: **30.05.2012**
(21) Application number: 10192395.1
(22) Date of filing: 24.11.2010
(51) Int. Cl.: D01D 5/00, D01F 1/10, D01D 5/38, A61L 17/08, A61L 27/24

(54) **Product of crosslinked material and method for producing the same**

(71) Applicant: SpinPlant GmbH, 04103 Leipzig (DE)
(72) Inventor: Ganey, Timothy, Tampa, FL 33604 (US); Meisel, Jörg, Berlin, 14163 (DE)
(74) Representative: Sokolowski, Fabian

(57) **Abstract**

The invention relates to a method for producing a nanofiber-based product, comprising the steps of providing a carrier material solution comprising a carrier material, and of bringing the carrier material in contact with a collector by electrospinning the carrier material solution out of a spinning device. Thereby, the collector has a first electrical polarity and the spinning device has a second electrical polarity being opposite to the first polarity. According to the invention, the carrier material essentially consists of a polymer being - at least after having contacted the collector - chemically crosslinked with the residue of a crosslinker and/or embedded in a polymer, which polymer is formed by the crosslinker. The crosslinker is chosen from the group consisting of glutaraldehyde, carbodiimide, genipin, photoreactive diazines and a compound according to the general formula (1) 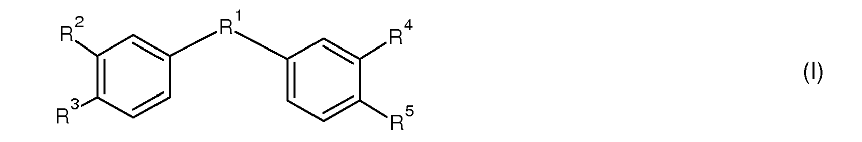 wherein wherein R¹ is a single bond between the adjacent carbon atoms, or a carbohydrate chain having 1 to 10 carbon atoms and optionally bearing a hydroxy group, the carbohydrate chain being saturated, unsaturated or polyunsaturated, and wherein R², R³, R⁴ and R⁵ are independently from each other a hydrogen; a carbohydrate chain having 1 to 10 carbon atoms and optionally bearing a hydroxy group, the carbohydrate chain being saturated, unsaturated or polyunsaturated; a hydroxy group; or a sulfhydryl group; with the provision that the compound bears at least two hydroxy groups, or two sulfhydryl groups, or one hydroxy group and one sulfhydryl group. The invention further relates to an according product and its use.

## Description

The invention relates to a method for producing a nanofiber-based product according to the preamble of claim 1, a product which can be obtained by such a method according to the preamble of claim 12 and the use of such a product according to claim 15.

It is known from prior art to electrospin different polymers into nanofibers. However, the stability of these nanofibers is often not satisfactory high. When the electrospun fibers are brought into contact with water or another solvent, dissolution of the fibers often occurs. Many approaches to stabilize the polymers by crosslinking have failed.

It is an object of the invention to provide a method by which highly stable nanofiber-based products can be obtained as well as to provide such nanofiber-based products.

This object is achieved by a method having the characteristics of claim 1. This method for producing a nanofiber-based product comprises the steps of providing a carrier material solution which itself comprises a carrier material, and bringing the carrier material in contact with the collector by electrospinning the carrier material solution out of the spinning device. Thereby, the collector has a first electrical polarity and the spinning device has a second electrical polarity which is opposite to the first polarity. According to the invention, the carrier material essentially consists of a polymer which is - at least after it has contacted the collector - chemically crosslinked with the residue of a crosslinker and/or embedded in a polymer formed by the crosslinker. The carrier material is preferably finally crosslinked and/or finally embedded already immediately after having contacted the collector, i.e. upon contact with the collector. In other words, the carrier material is solved or dispersed together with the crosslinker in the carrier material solution and is then precipitated from the carrier material solution under electric field strength onto the collector. Due to this precipitation, the crosslinking and/or embedding reaction ends. Alternatively, the crosslinker is added to the carrier material solution during electrospinning. Also in this case, crosslinking is terminated upon carrier material precipitation on the collector.

It turned out that electrospun fibers which had been crosslinked after electrospinning did not show an enhanced stability with respect to non-crosslinked nanofibers. Therefore, it is crucial that the crosslinking is finished upon formation of the nanofibers. This is presently the case when the carrier material contacts the collector, because carrier material and crosslinker are electrospun together.

It has been also turned out that not any generally known crosslinker can be used to successfully crosslink the polymer, but only a crosslinker which is chosen from the group consisting of glutaraldehyde, carbodiimide, genipin, photoreactive diazines and a compound according to the general formula (1) 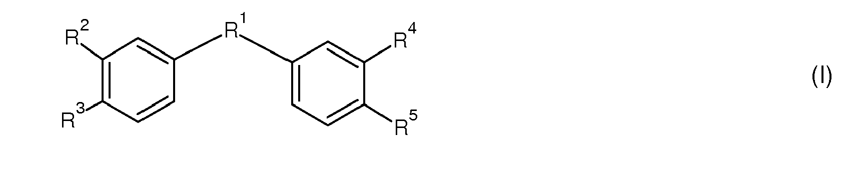 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group.

The term "crosslinker" is used in the entire text to encompass chemical substances which crosslink the carrier material by forming chemical bonds to the carrier material and/or by forming a polymer in which the carrier material is embedded. It is possible that chemical bond formation between crosslinker and carrier material also takes place in case of a polymer of crosslinker in which the carrier material is embedded. Thus, the carrier material can intercalate a polymer of a crosslinker and additionally can form chemical bonds to the crosslinker. Alternatively, only chemical bond formation (without polymeric embedding or intercalation) can take place. A substance comprising a polymer of a crosslinker and an embedded (or embedded and at least partially chemical linked) carrier material can also be denoted as composite or as nanofiber reinforced composite.

The crosslinker genipin has the following molecular structure:

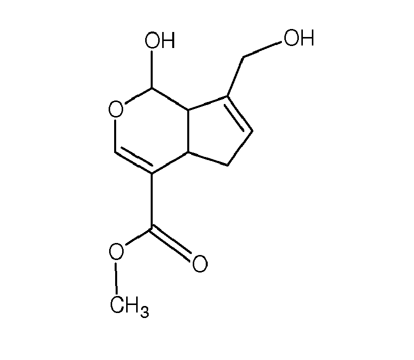

During electrospinning, a voltage difference between the electrified carrier material to be spun and the grounded collector exists. This voltage difference ameliorates crosslinking processes of the single polymer molecules of the carrier material when a crosslinker belonging to the above-explained group is used.

Well-suited examples for photoreactive diazines are photoreactive analogues to leucine and methionine.

In an embodiment R¹ is a residue having the following formula (II) 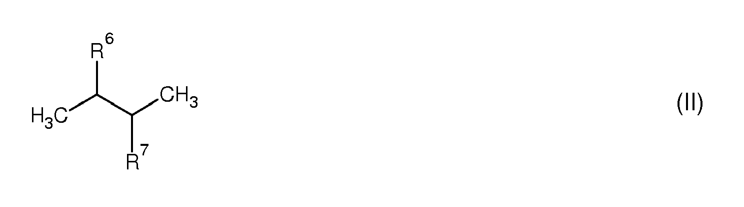 wherein each of the two indicated terminal methyl residues is linked to a carbon atom of each benzene ring and wherein R⁶ and R⁷ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group.

This means that in this embodiment the two benzene rings of the compound according to formula (I) are linked together by an optionally derivatized butyl chain, leading to a compound of the general formula (III):

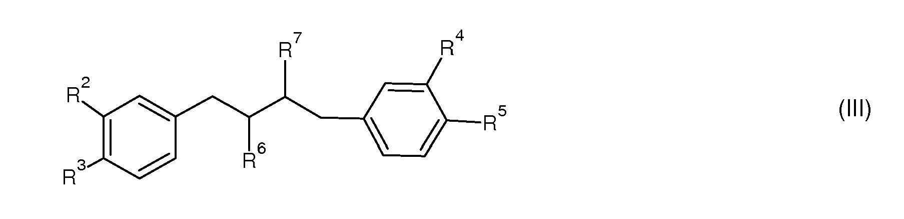

In an embodiment, at least two hydroxy groups or at least two sulfhydryl groups or at least one hydroxy group and one sulfhydryl group of the compound according to the general formula (I) are bound to a single or to both benzene rings of this compound. By binding the hydroxy and/or sulfhydryl groups to the benzene rings, a higher reactivity of the hydroxy and/or sulfhydryl group is achieved which leads to a better crosslinking performance of the according compound. The reactive groups (hydroxy and/or sulfhydryl) can be positioned in the benzene rings in ortho, meta or para position to each other, wherein an ortho position is preferred.

In a further embodiment, the crosslinker bears at least one, preferably at least two, preferably exactly two catechol groups. Thus, the crosslinker might be a di-catechol or an according derivative.

In a further embodiment, all hydroxy and/or sulfhydryl groups of the compound are bound to the benzene rings and no further such groups exist in the molecule.

The compound according to the general formula (I) mediates crosslinking of polymers particularly by its hydroxy or sulfhydryl groups. For example, an ether bridge can be formed by reaction of a hydroxy group of the compound according to the general formula (I) and the polymer. Alternatively, a disulfide bridge may be formed between the sulfhydryl group of the compound according to the general formula (I) and a sulfhydryl group of a polymer to be crosslinked. Since the compound according to the general formula (I) bears at least two reactive groups (hydroxy and/or sulfhydryl), it has at least two reactive centers so that long cross-linked networks can be produced. The more hydroxy and/or sulfhydryl groups the compound according to the general formula (I) bears, the higher branched can be the resulting crosslinked polymer.

The crosslinker might also react with only specific amino acids of the carrier material to be crosslinked if this carrier material bears amino acids. For example, the crosslinker might only react with lysine or arginine residues of the carrier material but not with other amino acid residues of the carrier material. The stiffer material properties following crosslinking with a crosslinker, in particular with NDGA, suggest that similar chemistries that are shown in cartilage may take place as well. It was previously described that advanced glycation end products of arginine and lysine contribute to stiffness, color, and an apparent reduction in both amino acids noted above. In a preferred embodiment, the crosslinker reacts with specific amino acids of the carrier material and additionally forms a polymer in which the carrier material can be embedded.

A possible polymerization scheme for polymer formation of the crosslinker is explained in the following. Assuming the crosslinker being a di-catechol derivative, 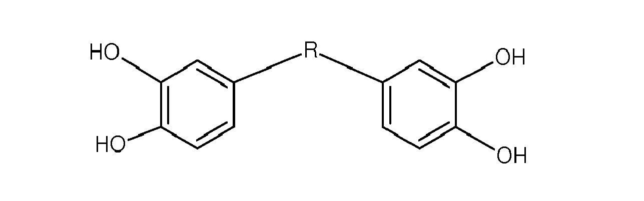 oxidation to a di-quinone takes place:

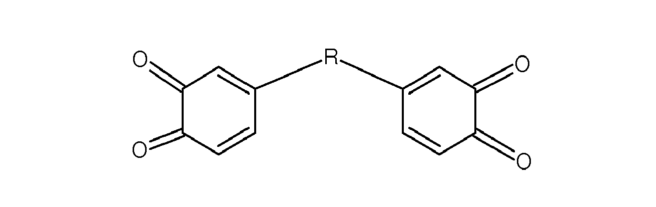

Two di-quinones react then via aryloxy free radical generation and subsequent oxidative coupling as well as reoxidation to a bisquinone:

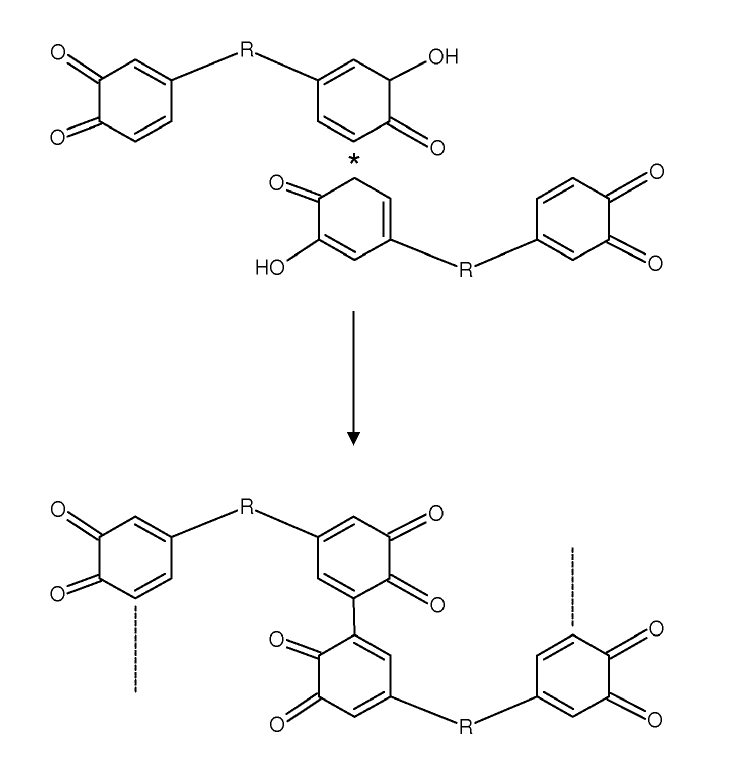

Further equivalent reactions lead to an extended polymer of bisquinone units so that a bisquinone polymer is formed. The carrier material can well intercalate this bisquinone polymer and can thus be embedded in it.

In an embodiment, the compound according to the general formula (I) bears more than three, in particular more than four, in particular more than five, in particular more than six, in particular exactly four reactive groups of the type hydroxy and/or sulfhydryl.

In a further embodiment, the crosslinker is nordihydroguaiaretic acid (NDGA). The structure of NDGA is the following:

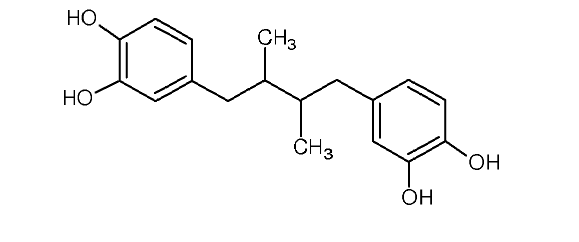

It turned out that NDGA is a very well suited crosslinker for crosslinking the polymer of the carrier material. Nanofibers of the carrier material being crosslinked by NDGA show a very high stability and are very long-lasting in solutions in which non-crosslinked polymers already dissolve. NDGA forms a polymer

In a further embodiment, the crosslinker is used in an amount of 2 to 20 wt % with respect to the dry mass of the polymer of the carrier material. Further suited amounts are 5 to 15 wt %, in particular 7 to 13 wt %, in particular 9 to 11 wt %, in particular around 10 wt %.

In a further embodiment, the carrier material is crosslinked before it reaches the collector. In doing so, cross-linking can take place particularly well not only on the surface of the nanofibers already being electrospun, but also internally. This enhances the stability of the nanofibers themselves and between each other. As explained above, it is crucial that the crosslinking is finished upon formation of the nanofibers. This is the case when the carrier material contacts the collector. If crosslinking is already finished a certain while before the carrier material contacts the collector, the integrity of the nanofibers is even more enhanced when the impact of the contact with the collector acts upon them.

In a further embodiment, the carrier material comprises of collagen, a mixture of collagen and hydroxy apatite, gelatin, alginates, chitosan, silk, cellulose, polyurethane, a polyester, polycaprolactone, polylactide, polypyrrole, polyaniline, polyacetylene, polythiophene, a copolymer of the preceding polymers and/or a copolymer bearing carboxylic acid groups and/or amine groups.

Further suited carrier materials are amino acid structures like oligopeptides or polypeptides. Oligopeptides are considered to consist of a sequence of up to 10 amino acids, whereas polypeptides are considered to be amino acid structures having more than 10 amino acids. Proteins are to be considered to be encompassed by the term "polypeptide". This means the amino acids structure can exhibit a primary, secondary and tertiary structure by itself so that the structure of the material to be deposited on the collector can exhibit an individually adjusted sub-structure. By using an amino acid structure, one can take advantage not only of the secondary or tertiary structure of the oligopeptides or polypeptides, but also of the amphiphilic charge of the single amino acids being present in the oligopeptides or polypeptides.

Well-suited collagens are collagen type I, II, III, V, or XI, wherein type I collagen is particularly well suited. The collagen might for example have a human, bovine, equine, ovine or fish origin or can be an artificial collagen resembling human, bovine, equine, ovine or fish collagen, or might consist of collagen, or collagen fibrils that have been expressed in culture by vector incorporation without limitation to mammalian source.

A particularly well-suited material is collagen. Using collagen as carrier material and NDGA as crosslinker leads to very well crosslinked and stable collagen nanofibers. They can be used in a particularly advantageous manner for producing a product according to the instant method.

The spinning device may be a nozzle through which the carrier material solution can be sprayed, or a turning (or rotating) pin located in a bath of the carrier material solution. By turning or rotating the pin in the bath, the carrier material solution is transported out of the bath and accelerated in the direction of the collector.

In an embodiment, the electrospinning may be performed at a voltage of 8 to 20 kV, in particular of 10 to 17 kV, in particular of 12 to 15 kV between the collector and the spinning device. A voltage of approximately 12.5 kV is particularly suited. Those voltages are well suited for accelerating the carrier material solution sufficiently fast out of the spinning device towards the collector. Further electrospinning parameters like the speed of a rotating pin for ejecting some of the carrier material solution out of a reservoir of the carrier material solution or the flow rate of the carrier material solution towards a nozzle, and the distance between the spinning device and the collector can be adjusted to the respective needs according to standard protocols of electrospinning.

In a further embodiment, the polymer is solved or dispersed in at least one liquid chosen from the group of water, alcohols like methanol or ethanol, aqueous solutions of acids or bases like acetic acid or sodium hydroxide and organic solvents like acetone or 1,1,1,3,3,3-hexaflouoro-2-propanol to produce a carrier material solution.

The term "carrier material solution" also encompasses "carrier material dispersions". This means, it is not necessary that the carrier material is ideally solved in an according liquid. If an essentially stable dispersion of the carrier material in an according liquid is established, electrospinning can also take place.

In a further embodiment, the carrier material is deposited onto the collector to form a sheet material. Such a sheet material can be used as bone substitute material or as wound-covering fleece.

In an alternative embodiment, the carrier material is removed from the collector after having contacted it and is then spun to a yarn. Such a yarn may be used as suture material in orthopaedic or surgical applications. The linear material, or yarn, or bioyarn, may also be embodied as a raw material characterized by its strength, stiffness, elasticity, modulus, charge and composition as to make it a linear material that can be braided, woven, knitted, plied, or in other ways converted to mesh, fabric, matted material, or laminated with specific endowment as to afford areas between the threads to dynamic as well as static properties that align to the degradation of the material and regeneration of the biologic tissue.

In a further embodiment, the electrospun carrier material is washed after having been removed from the collector. By washing, remainders of non-reacted crosslinker or reaction co-products can be removed, thus improving the biocompatibility of the obtained product. Washing can be performed by an aqueous solution of an alcohol like for example ethanol in a concentration of for example 80 %, 70 %, 60 % or 50 % (v/v). Further suited washing solutions are low salt buffers like phosphate buffered saline (PBS). Washing can be performed in a two-step manner by first using an alcoholic solution and afterwards a low salt buffer (or first a low salt buffer and afterwards an alcoholic solution) as washing solutions.

In an alternative embodiment, no washing step is necessary due to the specific crosslinker chosen and the concentration in which it is used.

The object is also achieved by a product which can in particular be obtained by a method according to the preceding explanations. Such product comprises a carrier material being built up from nanofibers having a diameter of less than 1 200 nm, in particular less than 1 100 nm, in particular less than 1 000 nm, in particular less than 900 nm, in particular less than 800 nm, in particular less than 700 nm, in particular less than 600 nm, in particular less than 500 nm, in particular less than 400 nm, in particular less than 300 nm.

According to the invention, the carrier material essentially consists of a polymer which is chemically crosslinked with the residue of a crosslinker. This crosslinker is chosen from the group consisting of glutaraldehyde, carbodiimide, genipin and photoreactive diazines as well as a compound according to the general formula (1) 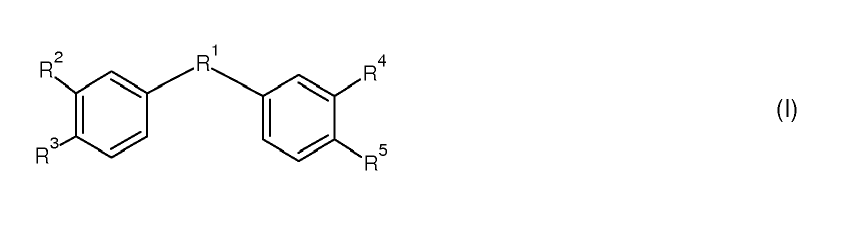 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group.

In an embodiment, the molecular ratio between the carrier material and the residue of the crosslinker is in the range of 20:1 to 5:1, in particular of 15:1 to 7:1, in particular of 12:1 to 8:1, in particular of 11:1 to 9:1 in the product.

In an embodiment, the product further comprises at least one auxiliary substance of the group consisting of osteoinductive substances, electrically conductive substances, electrically semiconductive substances, electrically insulating substances, antibacterial substances, antiviral substances, antifungal substances, ceramics (like, e.g., hydroxyapatite ceramics), barium, copper, bromine, niobium, lithium, germanium, titanium, lead, zirconium, silicon, silver, zinc, polyurethane and silver hydrogen sulfate, gallium orthophosphate (GaPO₄), langasite (La₃GaₛSiO₁₄), barium titanate (BaTiO₃), lead titanate (PbTiO₃), lead zirconate titanate (Pb[Zr*ₓ*Ti_{1-*x*}]O₃ 0<x<1), potassium niobate (KNbO₃), lithium niobate (LiNbO₃), lithium tantalate (LiTaO₃), sodium tungstate (Na₂WO₃), Ba₂NaNb₅O₅ and Pb₂KNb₅O₁₅, wherein the auxiliary substance is chemically and/or physically bound to the carrier material.

By such an auxiliary substance, additional biological and/or chemical effects can be introduced into the product. The auxiliary substance can be entrapped within the polymer network of the carrier material or the carrier material and the crosslinker, respectively. The entrapment offers the effect that a certain placement/precipitation of the auxiliary substance can be achieved, e.g. a placement/precipitation in the banding structure of the carrier material perpendicular to scaffold orientation. Thus, if hydroxyapatite is used as auxiliary substance and collagen is used as carrier material, the natural orientation of hydroxyapatite with respect to collagen in normal bone (i.e. in the banding structure of bone perpendicular to scaffold orientation) can be mimicked.

In an embodiment, the product exhibits an maximum tensile strength of ca. 50 to 200 MPa, in particular of ca. 75 to 175 MPa, in particular of ca. 90 to 150 MPa, in particular of ca. 100 to 125 MPa.

In a further embodiment, the product exhibits an elastic modulus of ca. 300 to 700 MPa, in particular of ca. 350 to 650 MPa, in particular of ca. 400 to 600 MPa, in particular of ca. 450 to 580 MPa, in particular of ca. 500 to 550 MPa.

Further embodiments explained with respect to the claimed method can also be applied in an analogous way to the claimed product and are not repeated here for the sake of brevity only.

A product having the characteristics as explained above can be very well used as scaffold for growing cells in vitro or in vivo or as suture material. The cells to be grown can be mesenchymal stem cells, fully competent stem cells, expanded somatic lineages, separated and suspended cells, peripheral circulating cells and cells of either included or induced potential. In vivo cell growth can be for example achieved with respect to bone growth or with respect to healing processes of wounds. Suture material made of a product as explained above can also ameliorate healing processes at the suture sites since cells attach more easily to such a suture material than to conventional suture materials.

For example, the sheet material can be implanted into a body of a patient (either human or animal) and act as (temporary) bone substitute material. If it is made from a biodegradable or bioresorbable material like for example collagen, the sheet material will be resorbed or degraded over time and newly grown bone will replace the sheet material step by step. By promoting bone growth due to the specific structure of the sheet material, healing processes after operations are accelerated. The use of a sheet material as described above for enabling tissue growth either in vivo or in vitro is also part of the invention. Specifically, the use of a sheet material as described above as bone substitute material is encompassed by the invention. The sheet material may embody itself with charge that potentiates tissue differentiation. Stem cell differentiation has been closely tied to electrovolt membrane potential during phenotypic emergence. Electrospinning, charge potentiation, and multi-laminar sheet formation all carry the option to defined physical conditions of the matrix, and define voltage differences in regenerative tissues. Processed materials from electrospun biologic components, both linear and in piled matte, will be used to define matrix charge and enliven the differentiation process.

## Claims

1. Method for producing a nanofiber-based product, comprising the following steps:
- providing a carrier material solution comprising a carrier material,
- bringing the carrier material in contact with a collector by electrospinning the carrier material solution out of a spinning device, the collector having a first electrical polarity and the spinning device having a second electrical polarity being opposite to the first polarity,
**characterized**
**in that** the carrier material essentially consists of a polymer being, at least after having contacted the collector, chemically crosslinked with the residue of a crosslinker and/or embedded in a polymer formed by the crosslinker, the crosslinker being chosen from the group consisting of a compound according to the general formula (1) 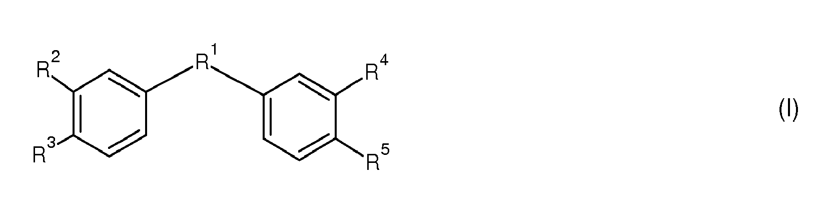 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group,
as well as of glutaraldehyde, carbodiimide, genipin and photoreactive diazines.

2. Method according to claim 1, **characterized in that** R¹ is a residue having the following formula (II) 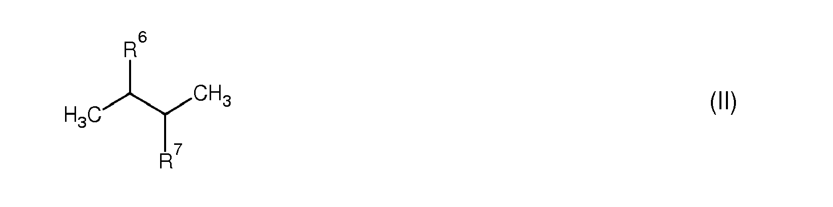 wherein each of the two indicated terminal methyl residues is linked to a carbon atom of each benzene ring and wherein R⁶ and R⁷ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group.

3. Method according to claim 1 or 2, **characterized in that** at least two hydroxy groups or at least two sulfhydryl groups or at least one hydroxy group and one sulfhydryl group of the compound according to general formula (I) are bound to the benzene rings of this compound.

4. Method according to any of the preceding claims, **characterized in that** the crosslinker is nordihydroguaiaretic acid.

5. Method according to any of the preceding claims, **characterized in that** the crosslinker is used in an amount of 2 to 20 percent by mass with respect to the dry mass of the polymer of the carrier material.

6. Method according to any of the preceding claims, **characterized in that** the carrier material is crosslinked before it reaches the collector.

7. Method according to any of the preceding claims, **characterized in that** the carrier material comprises collagen, a mixture of collagen and hydroxy apatite, gelatin, alginates, chitosan, silk, cellulose, polyurethane, a polyester, polycaprolactone, polylactide, polypyrrole, polyaniline, polyacetylene, polythiophene, a copolymer of the preceding polymers and/or a copolymer bearing carboxylic acid groups and/or amine groups, as well as oligopeptides or polypeptides.

8. Method according to any of the preceding claims, **characterized in that** the electrospinning is done at a voltage of 8 to 20 kV between the collector and the spinning device.

9. Method according to any of the preceding claims, **characterized in that** the polymer is solved or dispersed in at least one liquid chosen from the group of water, alcohols like methanol or ethanol, aqueous solutions of acids or bases like acetic acid or sodium hydroxide and organic solvents like acetone or 1,1,1,3,3,3-hexafluoro-2-propanol to produce a carrier material solution.

10. Method according to any of the preceding claims, **characterized in that** the carrier material is deposited onto the collector to form a sheet material.

11. Method according to any of claims 1 to 9, **characterized in that** the carrier material is removed from the collector after having contacted it and is then spun to a yarn.

12. Product, in particular obtainable by a method according to any of the preceding claims, comprising a carrier material being built up from nanofibers having a diameter of less than 1200 nm and being
**characterized**
**in that** the carrier material essentially consists of a polymer being chemically crosslinked with the residue of a crosslinker and/or being embedded in a polymer formed by the crosslinker, the crosslinker being chosen from the group consisting of a compound according to the general formula (1) 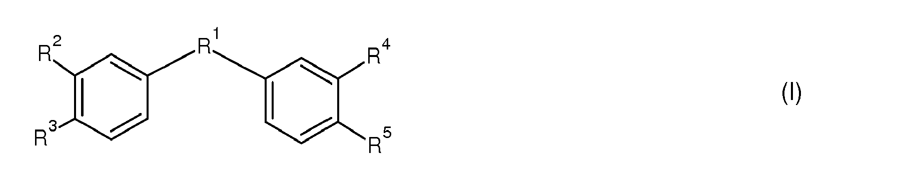 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group,
as well as glutaraldehyde, carbodiimide, genipin and photoreactive diazines.

13. Product according to claim 12, **characterized in that** the molecular ratio between the carrier material and the residue of the crosslinker is in the range of 20:1 to 5:1 in the product.

14. Product according to any of the preceding claims, **characterized in that** it further comprises at least one auxiliary substance of the group consisting of osteoinductive substances, electrically conductive substances, electrically semiconductive substances, electrically insulating substances, antibacterial substances, antiviral substances, antifungal substances, ceramics, barium, copper, bromine, niobium, lithium, germanium, titanium, lead, zirconium, silicon, silver, zinc, polyurethane and silver hydrogen sulfate, gallium orthophosphate, langasite, barium titanate, lead titanate, lead zirconate titanate, potassium niobate, lithium niobate, lithium tantalate, sodium tungstate, Ba₂NaNb₅O₅ and Pb₂KNb₅O₁₅, wherein the auxiliary substance is chemically and/or physically bound to the carrier material.

15. Use of a product according to any of claims 12 to 14 as scaffold for growing cells in vitro or as suture material.
